# EUROPEAN PATENT SPECIFICATION

(11) **EP 1 688 110 B1**
(45) Date of publication and mention of the grant of the patent: **11.11.2009**
(21) Application number: 03774232.7
(22) Date of filing: 25.11.2003
(51) Int. Cl.: A61F 13/02

(54) **BODY STICKING SHEET**
AM KÖRPER KLEBENDES TUCH
FEUILLE D'ADHESION AU CORPS

(43) Date of publication of application: 09.08.2006
(73) Proprietor: Sea Shell Co., Ltd., Osaka-shi, Osaka 543-0001 (JP)
(72) Inventor: KAWAHARA, Takemasa, Sea Shell Co., Ltd., Osaka-shi, Osaka 543-0001 (JP)
(74) Representative: Wolff, Michael
(86) International application number: PCT/JP2003/015055
(87) International publication number: WO 2005/051260

(56) References cited:
- EP-A2- 0 676 283
- JP-A- 6 339 495
- JP-A- 6 339 495
- JP-A- 61 257 644
- JP-A- 61 257 644

## Description

### Technical Field

The present invention relates to a body sticking sheet used to apply to a body for the purposes of assistance for weakened portions of muscle; and accelerations of cure and recovery of damages in muscle fibers, muscle spasm and the like.

### Background Art

When human being moves his (or her, and so forth) body in his daily life, sports and the like, his muscle is fatigued, whereby muscle affection, muscle spasm and the like are caused. When such tendency becomes remarkable, muscle pain arises, resulting in affections in respective regions in the body in addition to low back pain. Furthermore, there are causes of physical damages and affections derived from the case where strong muscle portions and weak muscle portions appear in the body; and unnecessary stresses are added to the weak portions. With respect to such physical damages and affections, a treatment for using a fixing tool and fixing an affected area with a bandage support, tape and the like to protect the affected area; and a treatment for suppressing inflammation with antiinflammatory analgesic plaster and icing to prevent deleterious changes in the affected area are adopted other than therapies.

On one hand, usual stiffness in shoulder or muscle pains being common problems in many peoples are considered to derive from such major causes due to hematogenous disorder or degradation of functions for removing fatigue products derived from decrease in frequency of usage (contraction motion in muscle) in muscle of a region concerned. As to stiffness in shoulder or muscle pains, such a treatment for moderating symptoms that an affected area is fixed with a tape, or that relaxation of the muscle is promoted by means of a magnetic therapeutic instrument, an antiinflammatory analgesic plaster and the like is executed.

Although a taping treatment is effective in both the cases as mentioned above, there may be a case where negative effects such as disorder of movement arise as a result of fixing over the peripheral portion of an affected area, whereby the motion of muscle is restricted so far as the taping tape or sheet is adequately applied. Furthermore, when an affected area is excessively fixed, induction of natural healing power is damaged so that a much time is required until reaching complete cure. Such problems may also be considered to be common in a bandage type supporter.

As a taping tape (including a sheet), a simple product obtained by applying only an adhesive on a backside of a cloth or a similar material having stretchability and a predetermined shape is commonly used. Heretofore, such type of a taping tape wherein the whole of the cloth or the similar material is made of the same raw material and in the same weaving manner, and thus all the area of the taping tape has an even degree of shrinkage. Among them, there is a product which is stickingly applicable to an area having a complicated configuration such as joints of a body without accompanying wrinkles (see Japanese Patent Application Laid-Open No. 2002-35196), or another product which is respondable to complicated motions in joints (see Japanese Patent Application Laid-Open No. 10-192337). However, these products are those having an even degree of shrinkage over the whole surface thereof, respectively.

In case of taping, it is considered to be desirable that a tape is stuck on an affected area so as to make muscle to be contracted. In these circumstances, for achieving the above-described ideal stuck conditions of a tape, it is required to adjust a state of tension in every region in the tape; and such correct treatment is difficult so far as a practitioner is a very skilled person.

When an ordinary person practices easily such taping, there is a danger of resulting in the disorder of movement or the degradation of natural healing power as mentioned above.

The present invention has been made under the conditions as mentioned above so that an object of the present invention is to provide a body sticking tape with which everybody can apply a proper taping in such that muscle is allowed to contract toward an affected area.

### Disclosure of the Invention

A body sticking sheet according to the present invention is characterized by including a portion with a small degree of shrinkage applied to an affected area, and a portion with a large degree of shrinkage around the former portion.

In case of taping the body sticking sheet, the portion with a small degree of shrinkage is stuck on the body in a condition wherein it is placed over the affected area. When the body sticking sheet is stuck on the body as described above, the portion with a large degree of shrinkage on the sticking sheet functions to make the muscle around the affected area to be contractive toward the side thereof.

The body sticking sheet of the present invention exhibits such function that the muscle around the affected area is allowed to contract toward the side thereof by merely sticking the sheet on a body portion with centering on the affected area without accompanying a particularly difficult practice. Accordingly, the body sticking sheet of the invention may decrease pains or damages in muscle, and further accelerate natural healing power to achieve early recovery of the damaged muscle, besides the body sticking sheet is also effective for assistance of a weak portion of muscle force.

### Brief Description of the Drawings

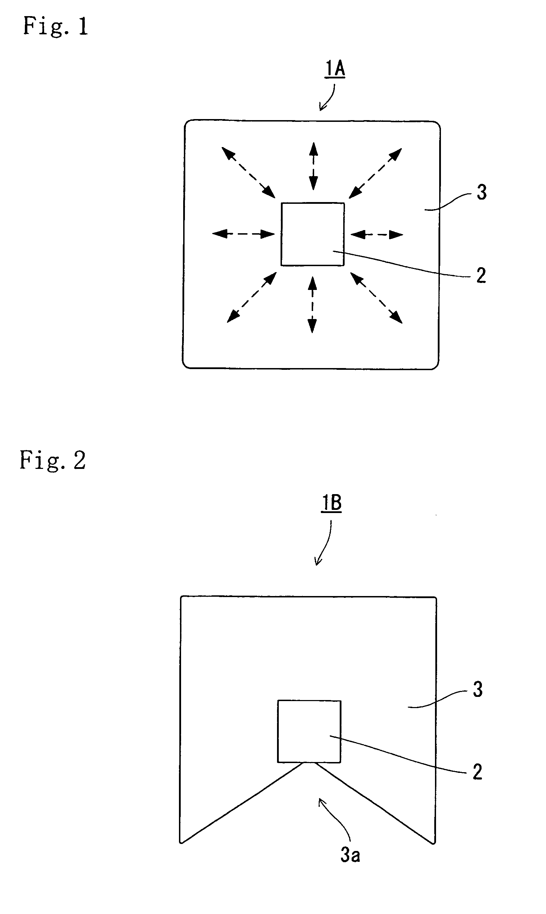

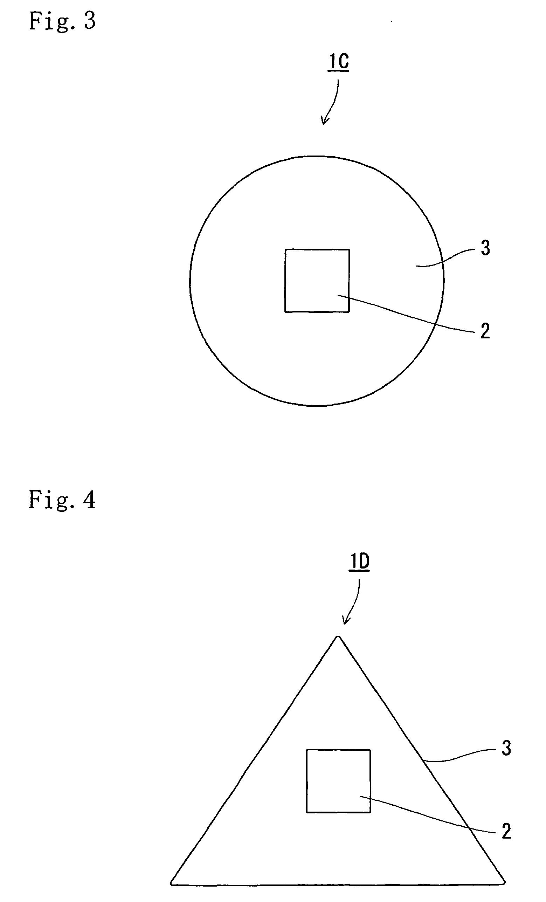

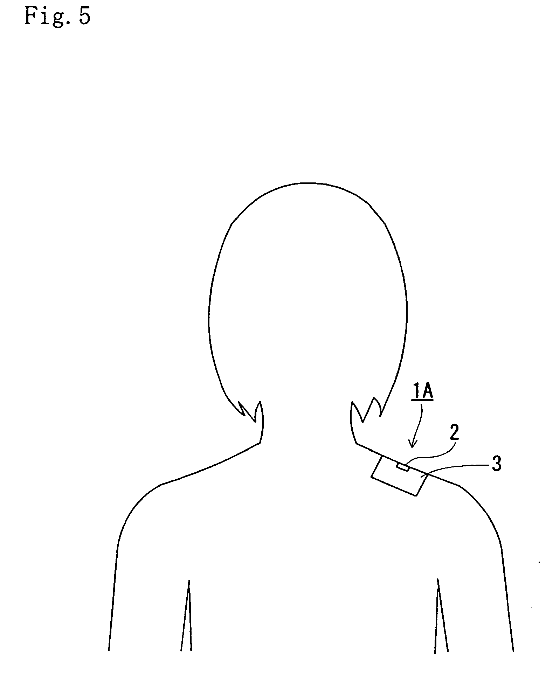

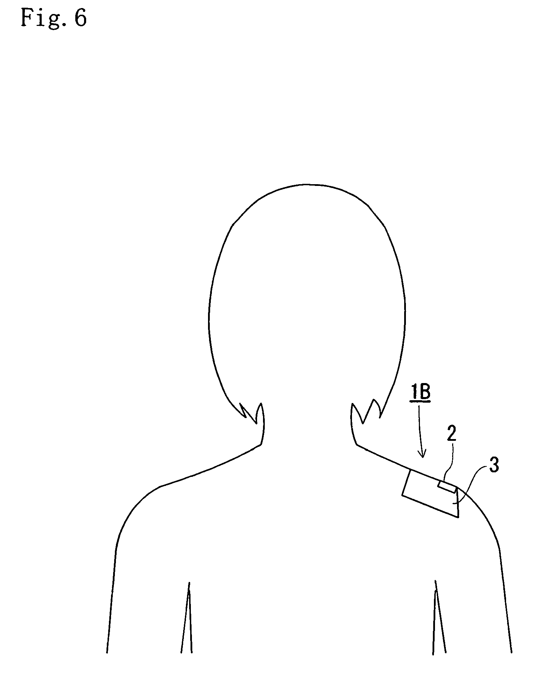

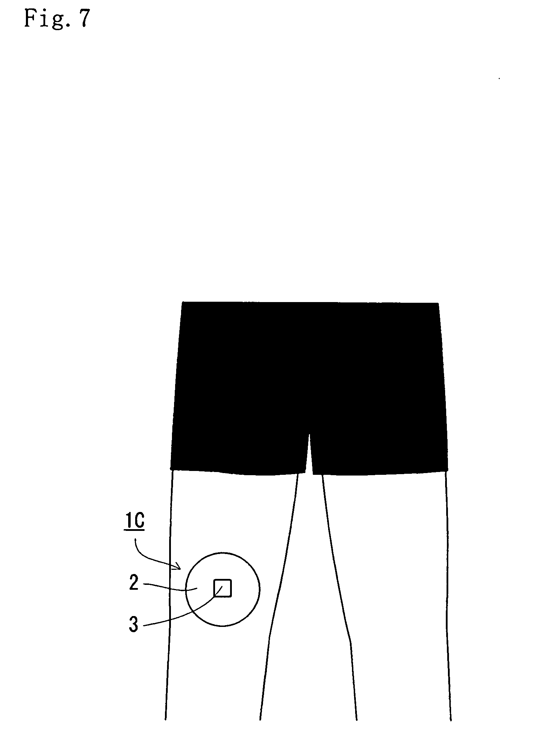

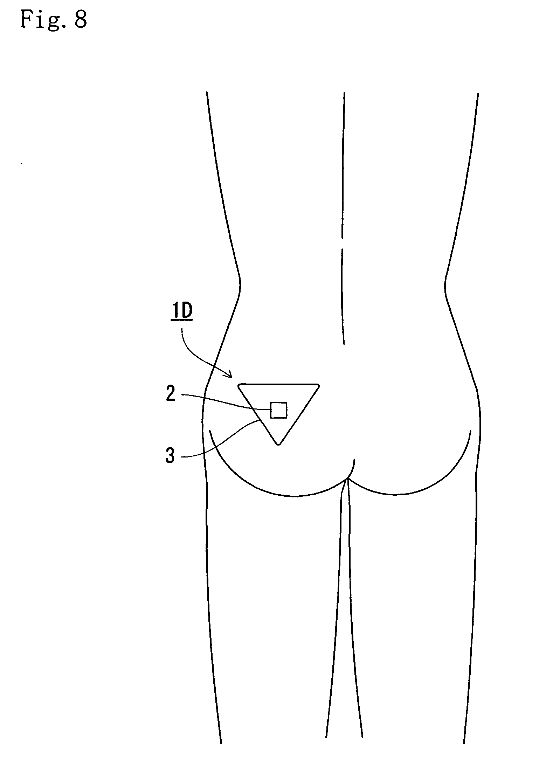

FIG. 1 is a diagram showing a first example (example 1) of the body sticking sheet; FIG. 2 is a diagram showing a second example (example 2) of the body sticking sheet; FIG. 3 is a diagram showing a third example (example 3) of the body sticking sheet; and FIG. 4 is a diagram showing a fourth example (example 4) of the body sticking sheet. Moreover, FIG. 5 is a view for showing an applied state of the body sticking sheet of the first example; FIG. 6 is a view for showing an applied state of the body sticking sheet of the second example; FIG. 7 is a view for showing an applied state of the body sticking sheet of the third example; and FIG. 8 is a view for showing an applied state of the body sticking sheet of the fourth example.

### Best Mode for Carrying Out the Invention

A variety of shapes of the body sticking sheet of the present invention in response to a body portion on which the body sticking sheet is to be stuck or symptoms in an affected area may be considered within a range including a basic configuration wherein a portion with a large degree of shrinkage is disposed around another portion with a small degree of shrinkage. FIGS. 1 to 4 show such examples of each modified one wherein reference characters 1A, 1B, 1C, and 1D designate body sticking sheets, 2 designates each of the portions with each small degree of shrinkage, and 3 designates each of the portions with each large degree of shrinkage.

Two types of raw materials having different degrees of shrinkage are combined to form the portion 2 with a small degree of shrinkage and the portion 3 with a large degree of shrinkage. It is preferred that a degree of shrinkage a of the portion 2 with a small degree of shrinkage is more than 0 %, not including just 0 %, while a degree of shrinkage b of the portion 3 with a large degree of shrinkage is 2 to 20 times higher than that of a. Due to a difference between the degrees of shrinkage in both the portions 2 and 3, for example, as shown in FIG. 1, the portion 3 with a large degree of shrinkage may be expanded and contracted radially (toward the directions represented by the arrows of dashed lines) with centering on the portion 2 with a small degree of shrinkage. It is preferred that a size of the portion 2 with a small degree of shrinkage is around 5 to 50 mm.

### (Example 1)

FIG. 5 illustrates an example wherein the body sticking sheet 1A shown in FIG. 1 is applied to chronic stiffness in the shoulder. A cause for stiffness in the shoulder is considered to be such that opportunities of attollens movement for bringing up the shoulder upwards decrease in working and a dairy life, whereby contraction of muscle such as cowl muscle, levator scapulae muscle, supraspinatus muscle, semispinalis capitis muscle, splenius capitis muscle and the like is not performed. As a result, a circulation of blood becomes worse, whereby a feed of oxygen and nutritional elements decrease, and the muscle itself scleroses, resulting in pains in muscle itself and compression of nerve, head ache and the like accompanied therewith.

When the body sticking sheet 1A is stuck on the portion of stiffness in the shoulder, contractive motion of the above-described muscles arises, whereby blood flow to an affected portion is promoted to feed sufficiently oxygen and nutritional elements, so that the stiffness in the shoulder is improved. Since a body region where the stiffness in the shoulder appears is positioned on both the front and rear sides between a substantially straight line shoulder line, it is preferred to use the rectangular body sticking sheet 1A shown in FIG. 1.

### (Example 2)

FIG. 6 illustrates an example wherein the body sticking sheet 1B shown in FIG. 2 is applied to a baseball shoulder. A cause for the baseball shoulder is considered to be decrease in muscular function of a muscle group (supraspinatus muscle, infraspinatus muscle, teres minor muscle, and subscapular muscle) called by the name of inner muscle. The inner muscle has a role to maintain stability of shoulder joints, and particularly it is required for motions (elevating and revolving an arm) of brachial part. When muscular functions of the inner muscle decreases to lose stability in shoulder joints, stresses are given to the joint portion by means of a muscle group exhibiting remarkable muscle contraction (greater pectoral muscle, cowl muscle, deltoid muscle, and latissimus dorsi muscle) called by the name of outer muscle, whereby inflammation cases in a synovial capsule for smoothening sliding of a tendon, so that pain appears.

When the body sticking tape 1B is stuck on an inner muscle having a small muscle amount, it becomes possible to elevate muscular functions of the inner muscle, whereby the shoulder joint can be stabilized. As a result, cooperativeness between the inner muscle and the outer muscle increases, whereby effects for preventing physical damages and improving mobility in a peripheral portion of the shoulder may be obtained. Since a region containing the inner muscle has an outline expanding outwards into a bowl-shaped, it is preferred to use a sticking sheet having a shape wherein the portion 3 with a large degree of shrinkage is provided with a notched portion 3a or an incision as in the body sticking sheet 1B shown in FIG. 2.

### (Example 3)

FIG. 7 illustrates an example wherein the body sticking sheet 1C shown in FIG. 3 is applied to muscle damage (muscle strain). The muscle damage means a state wherein muscle fibers are torn so that a damaged region of the muscle fibers bleeds. For the muscle damage, such treatment that the damaged region is iced, and then it is kept quiet in bed is usually taken. To keep the damaged region quiet in bed brings about regeneration of the muscle to cure the damaged region. On the other hand, however, there is a fear of resulting in a cause for secondary affection such as muscle induration and the like as a result of coagulation of blood bled. Furthermore, there is also such a problem that motion of the muscle is restricted, and further a term for recovery is prolonged.

When the body sticking sheet 1C is stuck on a body in such that the portion 2 with a small degree of shrinkage is applied over the damaged region, the muscle around the damaged region contracts toward the side of the damaged region, whereby early removal of the blood bled and pain producing substances is promoted due to a pressure derived from the contraction to assist regeneration of the muscle, and in addition, the damaged region is protected by the pressure of the contraction. In the case where a body sticking sheet is stuck on quadriceps muscle, it is preferred to use the body sticking sheet 1C shown in FIG. 3 having a circular outline in order to avoid fixing of the muscle over an unnecessary range to result in disturbance in gait.

### (Example 4)

FIG. 8 illustrates an example wherein the body sticking sheet 1D shown in FIG. 4 is applied to rupture of gluteal muscle group wherein the body sticking sheet having a triangular outline is used with taking characteristics of motion in the gluteal muscle group into consideration.

### Industrial Applicability

The body sticking sheet of the present invention is not limited to a taping product, but it is also applicable to a product on which an antiinflammatory analgesic plaster, or medicinal properties are applied.

## Claims

1. A body sticking sheet including two areas where the degrees of shrinkage are different, **characterized in that**
a portion (3) with a large degree of shrinkage is disposed around a portion (2) with a small degree of shrinkage applied to an affected area (Fig. 1, 2, 3, 4 ), and
the degree of shrinkage of said portion (3) with the large degree of shrinkage is 2 to 20 times higher than the degree of shrinkage of said portion (2) with the small degree of shrinkage,
wherein
the portion (3) with a large degree of shrinkage may be expanded and contracted radially with centering on the portion (2) with a small degree of shrinkage.

## Patentansprüche

1. Am Körper klebendes TUCH mit zwei Bereichen, in denen die Grade der Schrumpfung verschieden sind, **dadurch gekennzeichnet, dass** ein Teil(3) mit großem Schrumpfungsgrad um einen Teil(2) mit kleinem Schrumpfungsgrad, welcher in einem betroffenen Gebiet (Fig.1,2,3,4) anwendbar ist, herum angeordnet ist, und dass der Schrumpfungsgrad besagten Teiles(3) mit dem großen Schrumpfungsgrad zwei bis zwanzig Mal höher ist als der Schrumpfungsgrad besagten Teiles(2) mit dem kleinen Schrumpfungsgrad; wobei der Teil(3) mit dem großen Schrumpfungsgrad unter Zentrierung auf den Teil(2) mit kleinem Schrumpfungsgrad radial expandierbar und kontrahierbar ist.

## Revendications

1. Feuille adhérant au corps et comprenant deux zones dans lesquelles les degrés de retrait sont différents, **caractérisée en ce que**
une partie (3) ayant un grand degré de retrait est disposée autour d'une partie (2) ayant un petit degré de retrait appliqué à une zone affectée (figures 1, 2, 3, 4), et
le degré de retrait de la partie (3) ayant le grand degré de retrait est de 2 à 20 fois plus grand que le degré de retrait de la partie (2) ayant le petit degré de retrait,
dans laquelle
la partie (3) ayant le grand degré de retrait peut être agrandie et contractée radialement en se centrant sur la partie (2) ayant le petit degré de retrait.
